(19) Europäisches Patentamt

European Patent Office

Office européen des brevets

(11) **EP 1 199 029 B1**

(12) **EUROPÄISCHE PATENTSCHRIFT**

(45) Veröffentlichungstag und Bekanntmachung des Hinweises auf die Patenterteilung:
**25.10.2006 Patentblatt 2006/43**

(51) Int Cl.:
***A61B 5/0285*** (2006.01)

(21) Anmeldenummer: **01123616.3**

(22) Anmeldetag: **02.10.2001**

(54) **Vorrichtung zur Pulswellenlaufzeitbestimmung und extrakorporale Blutbehandlungseinrichtung mit einer solchen Vorrichtung**

Device for determining pulse wave passage time and an in-vitro blood treatment apparatus using such a device

Dispositif pour déterminer la fréquence du pouls et un appareil de traitement du sang in vitro utilisant un tel dispositif

(84) Benannte Vertragsstaaten:
**AT BE CH CY DE DK ES FI FR GB GR IE IT LI LU MC NL PT SE TR**

(30) Priorität: **19.10.2000 DE 10051943**

(43) Veröffentlichungstag der Anmeldung:
**24.04.2002 Patentblatt 2002/17**

(73) Patentinhaber: **Fresenius Medical Care Deutschland GmbH**
**61352 Bad Homburg v.d.H. (DE)**

(72) Erfinder: **Zhang, Wei, Dr.**
**97421 Schweinfurt (DE)**

(74) Vertreter: **Dreyhsig, Jörg**
**Fresenius Medical Care AG & Co. KGaA**
**Patent Department**
**Frankfurter Strasse 6-8**
**66606 St. Wendel (DE)**

(56) Entgegenhaltungen:
**EP-A- 0 911 044          WO-A-98/51211**
**US-A- 5 237 997**

- **DATABASE WPI Section PQ, Derwent Publications Ltd., London, GB; Class P31, AN 1989-218899 XP002210826 & SU 1 435 239 A (LE NI NEJROKHIRURG I), 7. November 1988 (1988-11-07)**
- **PATENT ABSTRACTS OF JAPAN vol. 1999, no. 09, 30. Juli 1999 (1999-07-30) & JP 11 089801 A (NIPPON COLIN CO LTD), 6. April 1999 (1999-04-06)**

EP 1 199 029 B1

**Beschreibung**

Die Erfindung betrifft das Gebiet der Bestimmung von Pulswellenlaufzeiten eines Patienten oder Spenders nach dem Oberbegriff des Anspruchs 1.

Der Blutdruck eines Patienten oder Spenders wird gewöhnlich mit Hilfe einer aufblasbaren Gummimanschette nach der Riva-Rocci-Methode gemessen. Diese Methode ermöglicht eine Messung nur zu definierten Zeitpunkten, bei denen der Druck der Manschette über einen gewissen Zeitbereich variiert wird. Eine kontinuierliche Messung ist daher auf Zeitintervalle, die durch die Meßmethode bedingt sind, begrenzt. Eine quasi-kontinuierliche Messung wäre jedoch mit einer ständig abwechselnden Expansion und Deflation der Gummimanschette verbunden, was mit für einen Patienten unzumutbaren Belastungen einhergehen würde.

Als Alternative zur nicht-invasiven Riva-Rocci-Methode ist die Methode der Pulswellenlaufzeitbestimmung bekannt, die ebenfalls nicht-invasiv durchgeführt werden kann. Dieser Methode liegt die Erkenntnis zugrunde, daß die Zeit, die eine durch eine Herzkontraktion eines Patienten oder Spenders erzeugte Pulswelle braucht, um durch das Gefäßsystem von einem ersten zu einem zweiten Ort zu gelangen, vom Blutdruck des untersuchten Individuums abhängt. Mißt man die Zeit, die zwischen dem zeitlichen Auftreten eines Herzschlags (detektiert z.B. mittels eines Elektrokardiogramms (EKG)) und dem Ankunftszeitpunkt der zugehörigen Pulswelle an einem vom Herzen entfernten Körperbereich (detektiert z.B. durch einen optischen Sensor am Ohrläppchen oder Finger) vergeht, so stellt diese Pulswellenlaufzeit ein direktes Maß für den Blutdruck des Patienten oder Spenders dar. Da die Pulswellenlaufzeit von Individuum zu Individuum schwankt, ist i.a. eine Kalibrierung mit Hilfe einer initialen Riva-Rocci-Messung notwendig. Eine Aussage über relative Änderungen kann jedoch direkt aus den relativen Änderungen der Pulswellenzeit erhalten werden. Der Zusammenhang zwischen dem Blutdruck und der Pulswellenlaufzeit ist weitgehend linear [Psychophysiology 13, 86 (1976)]. Da pro Herzschlag eine Messung möglich ist, stellt diese Meßmethode eine quasi-kontinuierliche Blutdruckmessung dar.

Die WO 89/08424 bzw. die DE 3 807 672 A1 beschreibt ein Meßverfahren zur Bestimmung der Pulswellenlaufzeit mit Hilfe eines EKGs und eines optoelektronischen Meßsensors an gut durchbluteten Hautgebieten. Da sich jedoch durch vasomotorische und andere Regelungen die Durchblutung des Hautgewebes und damit auch das Photostromprofil selbst mit der Zeit ändern können, ohne daß sich der Blutdruck notwendigerweise verändert hat, soll nach der initialen Kalibrierung nach der Riva-Rocci-Methode eine sich wiederholende Rekalibrierung allein anhand der Meßwerte des optoelektronischen Meßsensors anschließen. Dabei wird von einer für jedes Individuum konstanten Beziehung zwischen Pulswellenlaufzeit und Blutdruck ausgegangen. Die Rekalibrierung dient dem Zweck, aus den Photostrompulsprofilen auch zu späteren Zeitpunkten absolute Aussagen sowohl über den systolischen als auch über den diastolischen Druck zuzulassen.

Akute Notfälle z.B. während der Hämodialyse und/oder -filtration verlangen nach sofortigem Handeln. Eine Hauptkomplikation während einer solchen Blutbehandlung stellt der Abfall des Blutdrucks dar. Die häufigste Ursache für einen derartigen Zwischenfall ist eine Hypovolämie in Folge eines zu intensiven Flüssigkeitentzuges. Insbesondere während einer extrakorporalen Blutbehandlung ist es daher notwendig, den Blutdruck eines Patienten oder Spenders ständig zu überwachen, um mögliche Kreislaufkomplikationen frühzeitig zu erkennen.

Die EP-A-0 911 044, auf deren Offenbarung ausdrücklich Bezug genommen wird, beschreibt u.a. ein Hämodialyse- und/oder -filtrationsgerät, bei dem mit Hilfe der Messung der Pulswellenlaufzeit eine kontinuierliche Blutdruckbeobachtung unter gleichzeitig geringer Beeinträchtigung des Patienten ermöglicht wird. Anhand des Meßsignales der Pulswellenlaufzeit ist es möglich, kritische Blutdruckzustände früh zu erkennen und daraufhin das Bedienungspersonal umgehend zu informieren. Ggf. können automatisiert Gegenmaßnahmen am Hämodialyse- und/oder -filtrationsgerät z.B. durch Infusionen oder Konzentrationsveränderungen vorgenommen werden. Dieses bekannte Gerät geht aber - wie die Lehre nach der WO 89/08424 - von einer konstanten Beziehung zwischen Blutdruck und Pulswellenlaufzeit aus. Dies ist bei Blutbehandlungen, die insbesondere die Blutdichte verändern, eine nur unzureichend erfüllte Annahme. Insbesondere durch den Flüssigkeitsentzug während einer Hämodialyse- und/oder -filtrationsbehandlung nimmt die Blutdichte im Laufe der Behandlung zu (unter Blutdichte wird hier die Dichte von Blut als Flüssigkeit als solcher verstanden). Da die Blutdichte jedoch einen direkten Einfluß auf die Pulswellengeschwindigkeit und somit die Pulswellenlaufzeit hat, ergeben sich hierdurch ungenaue Meßwerte.

Der Erfindung liegt die Aufgabe zugrunde, eine Vorrichtung zur Pulswellenlaufzeitbestimmung eines Patienten oder Spenders derart weiterzubilden, daß die Veränderungen im Blutbild im Laufe der Zeit Berücksichtigung finden und damit eine genauere Überwachung des Blutdrucks ermöglicht wird.

Nach der Lehre der Erfindung wird diese Aufgabe durch eine Vorrichtung mit den Merkmalen des Anspruches 1 gelöst.

Die Erfindung baut auf die Erkenntnis auf, daß sich mit Hilfe von Messungen einer mit der Blutdichte korrelierenden Größe zum Zeitpunkt einer ersten Pulswellenlaufzeitmessung und zum Zeitpunkt einer zweiten Pulswellenlaufzeitmessung der Einfluß einer variablen Blutdichte zwischen den beiden Messungen kompensieren läßt. Auf diese Weise kann eine kompensierte erste oder zweite Pulswellenlaufzeit erhalten werden, die direkt mit der zweiten bzw.

ersten Pulswellenlaufzeit vergleichbar ist, als ob sie bei konstanter Blutdichte gemessen worden wären. Alarmzustände lassen sich auf diese Weise wesentlich verläßlicher anzeigen.

Die Geschwindigkeit, mit der sich eine Störung längs eines elastischen, zylindrischen und hinreichend langen Rohres in einer homogenen Flüssigkeit ausdehnt, beträgt [Y.C. Fung, in "Biomechanics-Circulation", 2. Edition, Springer, New York-Berlin, 1997, S. 140]:

$$c = \sqrt{\frac{A}{\rho}\frac{dp}{dA}} \qquad (1),$$

wobei

$\rho$: Dichte der Flüssigkeit,
A: Querschnittsfläche des Rohrs,
dA: Änderung der Querschnittsfläche,
dp: Änderung des Drucks im Rohr.

Nimmt man die Gültigkeit von Gleichung (1) für Blut in Arterien an, so ergibt sich für Blut der Dichte $\rho(t0)$ zum Zeitpunkt t0 im Vergleich zu Blut der Dichte $\rho(t)$ zum Zeitpunkt t bei konstantem Blutdruck $p(t0)$ für die Pulswellengeschwindigkeit c die Gleichung (2):

$$\frac{c(t, p(t0), \rho(t))}{c(t0, p(t0), \rho(t0))} = \sqrt{\frac{\rho(t0)}{\rho(t)}} \qquad (2).$$

Für die Pulswellenlaufzeit PTT, die das Durchlaufen der Pulswellen mit der Pulswellenausbreitungsgeschwindigkeit über eine definierte Strecke L angibt, erhält man einen ähnlichen Ausdruck:

$$\frac{PTT(t, p(t0), \rho(t))}{PTT(t0, p(t0), \rho(t0))} = \frac{L/c(t, p(t0), \rho(t))}{L/c(t0, p(t0), \rho(t0))} = \sqrt{\frac{\rho(t)}{\rho(t0)}} \qquad (3).$$

Mit Hilfe von Gleichung (3) ist es möglich, der Veränderung der Pulswellenlaufzeit durch eine Blutdichteänderung Rechnung zu tragen. Ist z.B. zu einem Zeitpunkt t0 eine erste Pulswellenlaufzeit PTT(t0,p(t0),ρ(t0)) und zu einem zweiten Zeitpunkt t eine zweite Pulswellenlaufzeit PTT(t,p(t),ρ(t)) gemessen worden, so kann mit Hilfe der Gleichung (3) der Einfluß der unterschiedlichen Blutdichte kompensiert werden. Jede der beiden Pulswellenlaufzeiten kann auf die Blutdichte der anderen Messung umgerechnet und damit vergleichbar gemacht werden:

$$PTT(t, p(t), \rho(t0)) = PTT(t, p(t), \rho(t))\sqrt{\frac{\rho(t0)}{\rho(t)}} \qquad (3a),$$

$$PTT(t0, p(t0), \rho(t)) = PTT(t0, p(t0), \rho(t0))\sqrt{\frac{\rho(t)}{\rho(t0)}} \qquad (3b).$$

Die für den Einfluß der Blutdichte kompensierten Angaben für die PTT lassen sich dann direkt vergleichen und auswerten. Ist zuvor eine Kalibrierung mit einem absoluten Blutmeßgerät vorgenommen worden, so sollten die Pulswellenzeiten auf die Blutdichte zum Zeitpunkt der Kalibrierungsmessungen umgerechnet werden.

Dadurch wird weiterhin eine genaue Umrechnung in absolute Blutdruckwerte möglich.

Die erfindungsgemäße Vorrichtung greift diese Erkenntnis auf. Eine Bestimmung einer mit der Blutdichte korrelierenden Größe ist dabei ausreichend, solange der Quadratwurzelterm in den Gleichungen (3a) oder (3b) zur Blutdichtekompensation bestimmt werden kann. Die Auswerteeinheit der erfindungsgemäßen Vorrichtung, die den Einfluß der Blutdichte auf die Pulswellenlaufzeit kompensiert, ist geeignet, eine Kompensation nach den Gleichungen (3a) oder (3b) durchzuführen.

Eine besonders bevorzugte Ausführungsform des Verfahrens kommt in einer Ausführungsform der erfindungsgemäßen Vorrichtung zum Einsatz, wobei die Mittel zur Bestimmung einer mit der Blutdichte korrelierenden Größe ein Meßgerät zur Bestimmung des relativen Blutvolumens oder der relativen Änderung des Blutvolumens umfassen. Unter der Annahme, daß die Dichteänderung nach Gleichung (3) nur durch Volumenveränderungen, aber nicht durch Masseveränderungen verursacht wurde, ergibt sich für den Wurzelterm aus Gleichung (3) mit den Volumina V(t0) und V(t):

$$\sqrt{\frac{\rho(t)}{\rho(t0)}} = \sqrt{\frac{m/V(t)}{m/V(t0)}} = \sqrt{\frac{V(t0)}{V(t)}} = \sqrt{\frac{V(t0)/V0}{V(t)/V0}} = \sqrt{\frac{RBV(t0)}{RBV(t)}} \qquad (4),$$

wobei V0 ein Vergleichsvolumen für die relativen Blutvolumina RBV ist. Nach Gleichung (4) ist dabei die Bestimmung der relativen Veränderung des Blutvolumens ausreichend, weitere Messungen zur Blutdichte oder absolute Angaben zum Blutvolumen sind nicht erforderlich.

In einer weiteren Ausführungsform der Erfindung stellt das Mittel zur Bestimmung einer mit der Blutdichte korrelierenden Größe ein Meßgerät zur Bestimmung des Hämatokrit (HCT) bzw. der relativen Hämatokritänderung dar. Nimmt man an, daß während der Meßzeit die Zahl der roten Blutkörperchen und deren Größe annähernd konstant bleiben, so ist die Hämatokritänderung umgekehrt proportional zur Blutvolumenänderung:

$$\frac{RBV(t0)}{RBV(t)} = \frac{HCT(t)}{HCT(t0)} \qquad (5).$$

Mit Hilfe der Gleichungen (4) und (5) läßt sich Gleichung (3) einfach in einen Ausdruck überführen, in dem neben der Pulswellenlaufzeitmessung nur noch die Angabe der relativen Hämatokritänderung HCT(t)/HCT(t0) erforderlich ist.

Die erfindungsgemäße Vorrichtung weist vorteilhafterweise ferner als Teil der Auswerteeinheit eine Auswertestufe auf, die die nach Gleichung (3a) oder (3b) kompensierten Werte anhand von vordefinierten Kriterien auf anormale Werte untersucht. Beispielsweise können einfache Alarmschwellenwerte, absolut oder relativ, gesetzt sein. Auch die Steigung der Pulswellenlaufzeit über der Zeit t kann ein Alarmkriterium darstellen. Schließlich - falls eine Kalibrierung mit einem absoluten Blutdruckmeßgerät erfolgt ist - können die Pulswellenlaufzeiten zunächst in einen absoluten Blutdruckwert umgerechnet und die Alarmkriterien auf diesen Wert angewendet werden.

Eine bevorzugte Ausführungsform der erfindungsgemäßen Vorrichtung beinhaltet eine Einrichtung zur Ermittlung eines EKG's. Die Auswerteeinheit ermittelt aus dem EKG den ersten Bezugszeitpunkt ta der Pulswellenlaufzeit PTT. Des weiteren ist an einer vom Herz entfernten Stelle eine Einrichtung zur Detektion der Pulswellen vorgesehen. Aus dem Signal dieser Einrichtung ermittelt die Auswerteeinheit den zweiten Bezugszeitpunkt te der Pulswellenlaufzeit PTT. In einer besonders bevorzugten Ausführungsform ist die Detektionseinrichtung ein Photoplethysmograph. Die Pulswellenlaufzeit PTT ergibt sich als Zeitspanne zwischen den beiden Bezugszeitpunkten (PTT=te-ta).

Bei einer besonders vorteilhaften Ausführungsform umfassen die Mittel zum Bestimmen der Pulswellenlaufzeit gleichzeitig die Mittel zur Bestimmung einer mit der Blutdichte korrelierenden Größe. So kann z.B. ein Photoplethysmograph gleichzeitig zur Hämatokritbestimmung herangezogen werden.

Die Auswerteeinheit kann auch Eingabe- und Ausgabefunktionen gegenüber dem Bedienungspersonal übernehmen, wie sie hinlänglich im Stand der Technik bekannt sind.

An dieser Stelle sei darauf hingewiesen, daß der Gedanke der beanspruchten Erfindung auch dahingehend umgesetzt werden kann, daß nicht die Pulswellenlaufzeit sondern direkt die Pulswellenausbreitungsgeschwindigkeit gemessen wird. Wie anhand von Gleichung (2) erkennbar ist, lassen sich die Abhängigkeiten der Meßwerte von der Blutdichte auch direkt auf die Pulswellenausbreitungsgeschwindigkeit übertragen, ohne daß von dem Kern der Erfindung abgewichen wird. Dieser Fall wird als äquivalente Realisierung der Erfindung angesehen.

Der Erfindung liegt auch die Aufgabe zugrunde, eine Blutbehandlungseinrichtung mit einem extrakorporalen Blutkreislauf und einer Vorrichtung zur Pulswellenlaufzeitbestimmung eines Patienten oder Spenders derart weiterzubilden, daß die Veränderungen im Blutbild im Laufe der Zeit Berücksichtigung finden und damit eine genauere Überwachung der Pulswellenlaufzeit und damit des Blutdrucks ermöglicht wird.

Nach der Erfindung wird diese Aufgabe durch eine Blutbehandlungseinrichtung nach dem Oberbegiff des Anspruches 16 dadurch gelöst, daß die Einrichtung eine Vorrichtung nach Anspruch 1 aufweist.

Wie bereits weiter oben ausgeführt wurde, ist insbesondere bei einer extrakorporalen Blutbehandlung eine ständige Beobachtung von Größen wie dem Blutdruck des Patienten oder Spenders hilfreich. Gleichzeitig wird bei diesen Blutbehandlungen zwangsläufig das Blutbild verändert. Insbesondere bei der Hämodialyse und/oder -filtration erfolgt auch eine Veränderungen des Blutvolumens. Diese Behandlungsformen, die die Funktionen der menschlichen Niere ersetzen oder zumindest ergänzen sollen, haben u.a. den Zweck, die Flüssigkeitsbilanz eines Patienten zu kontrollieren. Bei jeder Behandlung werden dem Patienten einige Liter Flüssigkeit während ca. 4-6 Stunden Behandlungszeit entzogen. Hierdurch kommt es zu einer beträchtlichen Veränderung der Blutdichte, auch wenn Flüssigkeit aus anderen Flüssigkeitkompartments des Körpers nachströmt.

Mit der Integration einer Vorrichtung nach Anspruch 1 in eine solche Blutbehandlungseinrichtung wird eine kontinuierliche und genaue Blutdruckmessung emlöglicht. Zusätzlich kann auf die Aktorik und Sensorik des bestehenden Gerätes zurückgegriffen werden. Wie bereits in der EP-A 0 911 044 beschrieben, kann die Einrichtung zur Detektion der Pulswellen an einer vom Herz entfernten Stelle einen Meßsensor umfassen, der bereits Teil der Blutbehandlungseinrichtung ist.

In einer vorteilhaften Ausführungsform der Erfindung handelt es sich hierbei um den arteriellen Drucksensor, d.h. den Drucksensor, der an der Blutzuführleitung zu einer Blutbehandlungseinheit angebracht ist.

Im Stand der Technik sind auch andere Sensoren bekannt, mit deren Hilfe Blutvolumen- oder Hämatokritänderungen extrakorporal bestimmt werden. In der EP-A 0 358 873 wird ein System zur Ultraschalllaufzeitbestimmung beschrieben, daß aus den Ultraschalllaufzeiten die relative Änderung des Blutvolumens und/oder des Hämatokrits berechnet. Es sind auch optische Verfahren bekannt, die im optisches Durchlichtverfahren die Hämoglobinkonzentration an der extrakorporalen Blutzuführteitung bestimmen, auf deren Grundlage wiederum der Hämatokrit- und die relativen Blutvolumina abgeleitet werden. Ein solches Verfahren ist z.B. Gegenstand der WO 94/27495. Eine Kombination von einem optischen Durchlicht- mit einem Streuverfahren, bei dem auch nur eine Lichtwellenlänge verwendet zu werden braucht, schlägt die WO 00/33053 vor.

Weitere Einzelheiten und Vorteile der Erfindung werden anhand von in den Zeichnungen dargestellten Ausführungsbeispielen näher beschrieben. Es zeigen:

Figur 1: Eine schematische Übersicht über eine erfindungsgemäße Vorrichtung zur Anwendung des Verfahrens zur Pulswellenlaufzeitbestimmung im Sinne eines unabhängigen Monitorgerätes;

Figur 2: Eine schematische Übersicht über eine extrakorporale Blutbehandlungseinrichtung bestehend aus einer Hämodialysevorrichtung mit einer Vorrichtung nach Figur 1.

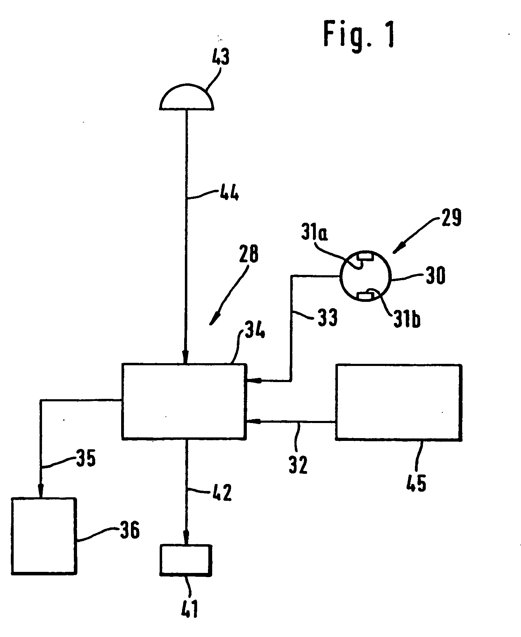

Das in Figur 1 dargestellte Meßgerät 28 zur Pulswellenlaufzeitbestimmung eines Patienten oder Spenders weist einen Elektrokardiographen 45, ein als Druckmanschette ausgebildetes absolutes Blutdruckmeßgerät 43, einen Photoplethysomograph 29 sowie eine Auswerteeinheit 34 auf. Alle Sensorkomponenten sind mit entsprechenden Leitungen 32, 33 und 44 mit der Auswerteeinheit verbunden. Der Elektrokardiograph 45 ermittelt anhand nicht näher dargestellter Elektroden die Spannungssignale, die sich an der Körperoberfläche eines Patienten oder Spenders durch die Herzerregung einstellen (EKG). Diese Signale werden über die Leitung 32 der Auswerteeinheit 34 zugänglich gemacht. Diese ermittelt aus der Lage der R-Zacke den ersten Bezugszeitpunkt ta für die Pulswellenlaufzeitbestimmung.

Der Photoplethysmograph 29 weist einen Sensor 30 auf, der aus einer infraroten Lichtquelle 31a und einem Photodetektor 31b besteht. Die Lichtquelle und der Photodetektor sind bei diesem Ausführungsbeispiel derart gestaltet, daß drei LED's und drei Photodioden Messungen bei den drei Wellenlängen 805 nm, 970 nm und 1310 nm ermöglichen. Ein solcher Photoplethysmograph ist in der WO 94/23643 beschrieben, auf deren Offenbarung hier ebenfalls ausdrücklich Bezug genommen wird.

Der Photoplethysmograph 29 wird an einem Körperteil, vorzugsweise an einem Finger oder Ohrläppchen des Patienten oder Spenders, derart befestigt, daß das Licht das Körperteil zumindest teilweise durchdringt, bevor es auf die Photodioden 31b fällt. Dies kann in einer Durchlichtanordnung, prinzipiell aber auch in einer Streulichtanordnung geschehen. Die Meßsignale werden über die Leitung 33 an die Auswerteeinheit 34 gegeben, die Mittel umfaßt, aus dem Kurvenverlauf den zweiten Bezugszeitpunkt te der Pulswellenlaufzeit zu bestimmen. Dies kann z.B. nach den in der EP-A 0 911 044 genannten Verfahren erfolgen. Dafür ist zunächst nur die Messung bei einer Wellenlänge erforderlich. Pulswellen verursachen in den Blutgefäßen eine Dehnung der Gefäße, was zu einer veränderten Absorption durch die veränderte Menge Blut und damit auch der Blutinhaltsstoffe führt. Bei dem beschriebenen 3-Wellenlängen-Photoplethysmographen trifft dies auf alle drei Wellenlängen 805 nm, 970 nm und 1310 nm zu, wobei die Messungen bei den ersten beiden Wellenlängen empfindlich auf die Stoffe Hämoglobin und Oxyhämoglobin sind und die Messung bei der dritten Wellenlänge die Plasmawasserabsorption betrifft.

Das absolute Blutdruckmeßgerät 43 kann eingesetzt werden, um Pulswellenlaufzeitmessungen zu kalibrieren. Nachfolgende Pulswellenwellenlaufzeitmessungen können dann durch die Auswerteinheit 43 direkt in absolute Blutdruckangaben umgerechnet und - falls erwünscht - angezeigt werden. Zu genaueren Angaben wird ebenfalls auf die EP-A 0 911 044 verwiesen.

Für eine blutdichtekompensierte Pulswellenlaufzeitmessung arbeitet das Meßgerät 28 wie folgt: Zu einem ersten Zeitpunkt t0 ermittelt die Auswerteinheit 34 aus einem eingehenden EKG-Signal (erster Bezugszeitpunkt ta) und einem nachfolgenden Pulssignal des Photoplethysmographen 29 (zweiter Bezugszeitpunkt te) eine erste Pulswellenlaufzeit PTT(t0,p(t0),p(t0)). (Da die PTT-Werte ($\approx$ 0,15...0,3 s) klein gegenüber den Zeiträumen aufeinanderfolgender PTT-Messungen sind, die signifikante Blutdruckänderungen betreffen, ist es unerheblich, ob für t0 entweder ta, te oder ein Zeitpunkt zwischen diesen beiden Zeitpunkten gewählt wird.) Gleichzeitig wird mit Hilfe des Photoplethysmographen 29 der Hämatokrit des Blutes des Patienten oder Spenders ermittelt. Hierzu werden Absorptionmessungen bei allen drei genannten Wellenlängen durchgeführt und wie in der WO 94/23643 beschrieben ausgewertet. Daraufhin wird ein absoluter Wert für den Hämatokrit HCT(t0) zum Zeitpunkt t0 erhalten.

Die Initiierung dieses Zeitpunktes t0 kann durch ein selbsttätig ablaufendes Programm oder durch ein Signal von außen - manuell oder über eine Schnittstellenverbindung - verursacht sein. Gleiches gilt für die Initiierung einer zweiten Messung zu einem Zeitpunkt t mit t>t0, bei dem die Pulswellenlaufzeit PTT(t,p(t),$\rho$(t)) sowie ein entsprechender Wert HCT(t) gemessen werden.

Die Mittel zur Blutdichtekompensation der Pulswellenlaufzeit in der Auswerteinheit 34 berechnen nun anhand der Gleichungen (3a) bzw. (3b) sowie (4) und (5) die blutdichtekompensierte Pulswellenlaufzeit PTT(t,p(t),$\rho$(t0)) bzw. PTT(t0,p(t0),$\rho$(t)).

Der erhaltene Wert kann dann direkt oder nach Umrechnung in einen Blutdruckwert, falls eine Kalibrierung mit Hilfe des absoluten Blutdruckmeßgerätes 43 erfolgt war, auf einer Anzeigeeinheit 36, die mit der Auswerteinheit 34 über eine Leitung 35 verbunden ist, angezeigt werden. Zusätzlich können Alarmgeber 41 vorgesehen sein, die mit einer Leitung 42 mit der Auswerteinheit 34 verbunden sind. Diese sind geeignet, akustische oder optische Alarmsignale abzugeben, sollten die Auswerteinheit 34 ein entsprechendes Signal hierzu geben. Dies geschieht dann, wenn die Auswerteinheit anhand des erhaltenen Blutdruck- oder Pulswellenlaufzeitwertes einen anormalen Zustand manifestiert, z.B. beim Unter- oder Überschreiten eines Schwellwertes oder bei einer zu schnellen Veränderung des Wertes innerhalb kurzer Zeit.

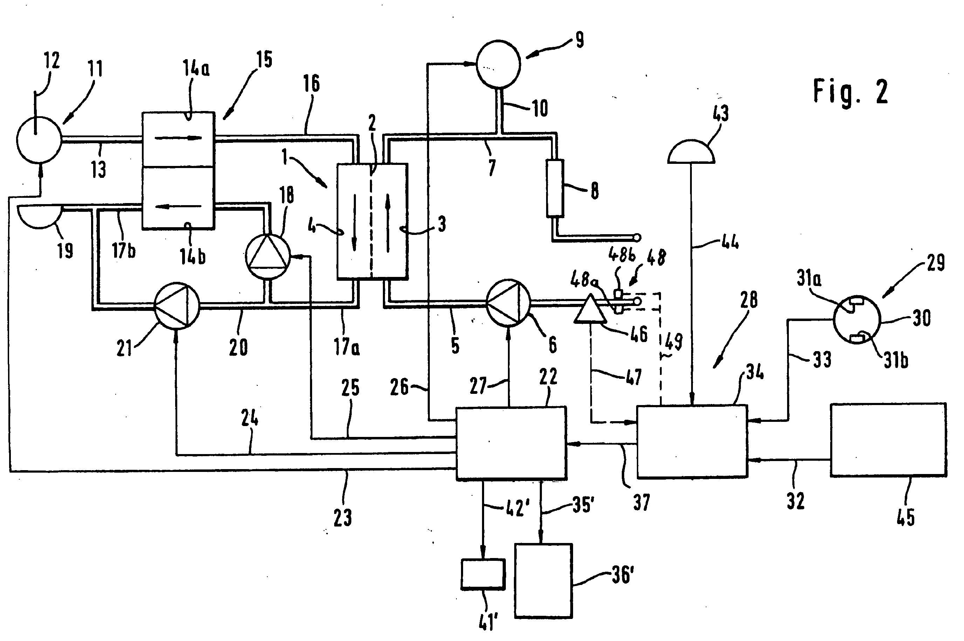

In Figur 2 ist eine Blutbehandlungseinrichtung mit einem Hämodialysator als Blutbehandlungseinheit dargestellt. Diese Apparatur entspricht in etwa der in der EP-A 0 911 044 erläuterten Hämodialysevorrichtung. Hier seien dennoch die wesentlichen Komponenten kurz erläutert: Die Blutbehandungseinrichtung weist einen Hämodialysator 1 auf, der durch eine semipermeable Membran 2 in eine Blutkammer 3 und eine Dialysierflüssigkeitskammer 4 getrennt ist. Der Einlaß der Blutkammer ist mit einem Ende einer Blutzuführleitung 5 verbunden, in die eine Blutpumpe 6 geschaltet ist, während der Auslaß der Blutkammer 3 mit einem Ende einer Blutabführleitung 7 verbunden ist, in die eine Tropfkammer 8 geschaltet ist. Der extrakorporale Blutkreislauf weist ferner eine Einrichtung 9 zur automatischen. Applikation einer Infusion, insbesondere einer physiologischen NaCl-Lösung (typischerweise 200ml) oder auch on-line-filtrierter Substituatlösung mit einer Substitutionsrate von typischerweise 150 ml/min auf. Die Infusion, die zumeist bolusartig folgt, wird über eine Zuführleitung 10, die stromauf der Tropfkammer 8 an die Blutabführleitung 7 angeschlossen ist, dem Patienten zugeführt.

Das Dialysierflüssigkeitssystem der Hämodialysevorrichtung umfaßt ferner eine Einrichtung 11 zur Aufbereitung der Dialysierflüssigkeit, wobei unterschiedliche Zusammensetzungen der Dialysierflüssigkeit (Elektrolytgabe) vorgegeben werden können. Die Dialysierflüssigkeitsaufbereitungseinrichtung 11 verfügt über eine Temperiereinheit 12, mit der die Temperatur der Dialysierflüssigkeit auf verschiedene Werte eingestellt und konstant gehalten werden kann. Sie ist über den ersten Abschnitt 13 einer Dialysierflüssigkeitszuführleitung mit dem Einlaß der ersten Kammerhälfte 14a einer Bilanziereinrichtung 15 verbunden. Der zweite Abschnitt 16 der Dialysierflüssigkeitszuführleitung verbindet den Auslaß der ersten Bilanzierkammerhälfte 14a mit dem Einlaß der Dialysierflüssigkeitskammer 4. Der Auslaß der Dialysierflüssigkeitskammer 4 ist über den ersten Abschnitt 17 einer Dialysierflüssigkeitsabführleitung mit dem Einlaß der zweiten Bilanzierkammerhälfte 14b verbunden. In den ersten Abschnitt 17a der Dialysierflüssigkeitsabführleitung ist eine Dialysierflüssigkeitspumpe 18 geschaltet. Der Auslaß der zweiten Bilanzierkammerhälfte 14b ist über den zweiten Abschnitt 17b der Dialysierflüssigkeitsabführleitung mit dem Auslauf 19 verbunden. Stromauf der Dialysierflüssigkeitspumpe 18 zweigt von der Dialysierflüssigkeitsabführleitung 17 eine Ultrafiltratleitung 20 ab, die ebenfalls zu dem Auslauf 19 führt. In die Ultrafiltratleitung 20 ist eine Ultrafiltrationspumpe 21 geschaltet.

Die Hämodialysevorrichtung umfaßt ferner eine zentrale Steuereinheit 22, die über Steuerleitungen 23 bis 27 mit der Blutpumpe 6, der Dialysierflüssigkeitspumpe 18, der Ultrafiltrationspumpe 21, der Einrichtung 11 zur Aufbereitung der Dialysierflüssikeit und der Einrichtung 9 zur automatischen Applikation eines Bolus verbunden ist.

Während der Hämodialysebehandlung wird die Blutkammer 3 von dem Blut des Patienten und die Dialysierflüssigkeitskammer 4 des Dialysators 1 von der Dialysierflüssigkeit durchströmt. Da die Bilanziereinrichtung 15 in den Dialysierflüssigkeitsweg geschaltet ist, kann nur soviel Dialysierflüssigkeit über die Dialysierflüssigkeitzuführleitung 16 zufließen, wie Dialysierflüssigkeit über die Dialysierflüssigkeitabführleitung 17 abfließen kann. Mit der Ultrafiltrationspumpe 21 kann dem Patienten Flüssigkeit entzogen werden.

Die Hämodialysevorrichtung weist darüber hinaus eine Vorrichtung 28 zur kontinuierlichen Pulswellenlaufzeitbestimmung gemäß Figur 1 auf. Die Bezugzeichen dieser Komponenten sind die gleichen wie in Figur 1. Der Alarmgeber 41 und die Anzeigeeinheit 36 werden jedoch in dem vorliegenden Fall praktischerweise durch die ohnehin vorhandenen derartigen Elemente 41' und 36' zusammen mit den Steuerleitungen 42' und 35' der Blutbehandlungseinrichtung dargestellt, die mit der Steuereinheit 22 verbunden sind. Die Auswerteeinheit 34 ist des weiteren über eine Leitung 37 mit der Steuereinheit 22 verbunden. Beide Einheiten könnten in der Tat physikalisch getrennte Einheiten darstellen, sie können aber auch in einer gemeinsamen Einheit, praktischerweise der Steuereinheit der Blutbehandlungsvorrichtung, vereint sein. Der Trennung kommt dann nur eine funktionelle Bedeutung zu.

Die Funktionsweise der Vorrichtung 28 ist bereits erläutert worden. Bei der Hämodialysevorrichtung nach Figur 2 erhält die Steuereinheit 22 nun ebenfalls die blutdichtekompensierten Meßwerte der Auswerteeinheit 34. Gemäß der hinterlegten Alarmkriterien kann die Steuereinheit 22 Gegenmaßnahmen vorschlagen oder ggf. automatisch durchführen, um einem erkannten kritischen Blutdruckzustand entgegenzuwirken.

Durch die während der Hämodialysebehandlung erfolgte Ultrafiltration werden dem Patienten beträchtliche Flüssigkeitsmengen entzogen, die zu einem Blutdruckabfall (Hypotension) führen können. Durch eine quasi-kontinuierliche Messung des Blutdrucks durch die Pulswellenlaufzeitmethode (ca. 1 Messung pro Sekunde) läßt sich eine hypotensive Phase bereits frühzeitig erkennen, bevor bei dem Patienten spürbare Symptome auftreten. Durch die Auswerteeinheit 34 werden diese Meßwerte nun mit gesteigerter Genauigkeit ermittelt, da der Einfluß der sich durch die Ultrafiltration ändernden Blutdichte kompensiert wird. Ein Abfall des relativen Blutvolumens um 20% sind keine Seltenheit während der Hämodialyse. Nach den Gleichungen (3) und (4) erhält man durch die Kompensation der Blutdichteänderung einen ca. 10% genaueren Meßwert für eine spätere Messung der Pulswellenlaufzeit für einen Vergleich mit einer früheren Messung.

Beispielhafte Gegenmaßnahmen gegen einen Blutdruckabfall können durch die Steuereinheit durch eine Veränderung der Elektrolytkonzentration über die Steuerleitung 23, durch eine Initierung einer Infusion über die Steuerleitung 26, durch ein Vermindern oder sogar Abschalten der Ultrafiltration über die Leitung 24 oder sogar durch einen sofortigen Stop der Behandlung durch Anhalten der Blutpumpe 6 eingeleitet werden.

In einer weiteren Ausführungform der Erfindung wird ein ohnehin meist vorhandener arterieller Drucksensor 46 an der Blutzuführleitung 5 dazu verwendet, den zweiten Bezugszeitpunkt te für die Pulswellenlaufzeitmessung zu erfassen. In diesem Fall ist der Drucksensor 46 über eine Leitung 47 mit der Auswerteeinheit 34 verbunden. Bei dieser Ausführungsform ist der Photoplethysmograph 29 bzgl. dieser Funktion entbehrlich.

Bei einer besonders vorteilhaften Ausführungsform ist der Photoplethysmograph 29 insgesamt entbehrlich. In diesem Fall sind die Mittel zur Bestimmung einer mit der Blutdichte korrelierenden Größe ebenfalls extrakorporal vorgesehen. Hierzu kann ein Blutvolumenmonitor 48 in der in der EP-A 0 358 873 beschriebenen Art an der Blutzuführleitung 5 befestigt sein. Dieser Blutvolumenmonitor besteht aus einem Ultraschallsender 48a und einem Ultraschallempfänger 48b, die die Laufzeit des Ultraschallsignals durch die Blutzuführleitung bestimmen. Über eine Leitung 49 ist der Blutvolumenmonitor 48 mit der Auswerteinheit 34 verbunden, die aus den Signalen ein Änderung der relativen Blutvolumens oder des Hämatokrits zwischen den Zeiten t0 und t ermittelt. Es ist auch die Anwendung anderer extrakorporaler Sensoren denkbar, die zum Beispiel den Hämatokrit optisch oder anhand anderer Meßgrößen an der Blutzuführleitung bestimmen.

Die Übernahme von Funktionen in den extrakorporalen Kreislauf kann unterschiedlich aufgeteilt sein. So mag es für bestimmte Situationen sinnvoll sein, die Mittel zur Bestimmung einer mit der Blutdichte korrelierenden Größe in den extrakorporalen Kreislauf zu integrieren, die Mittel zum Bestimmen der Pulswellenlaufzeit aber vollständig direkt am Körper des Patienten zu belassen.

**Patentansprüche**

1. Vorrichtung zur Pulswellenlaufzeitbestimmung eines Patienten oder Spenders mit
   Mitteln (29, 45) zum Bestimmen der Pulswellenlaufzeit von sich über das Gefäßsystem des Patienten oder Spenders fortpflanzenden Pulswellen, die durch dessen Herzkontraktionen erzeugt werden, zum Zeitpunkt t0 einer ersten Pulswellenlaufzeitmessung und zum Zeitpunkt t einer zweiten Pulswellenlaufzeitmessung,
   **dadurch gekennzeichnet, daß**
   Mittel (29) zur Bestimmung einer mit der Blutdichte korrelierenden Größe zu den Zeitpunkten t0, t der ersten und zweiten Pulswellenlaufzeitmessung und
   eine Auswerteeinheit (34), die den Einfluß der variablen Blutdichte auf die erste oder zweite Pulswellenlaufzeitmessung mit Hilfe der Messungen der mit der Blutdichte korrelierenden Größe zu den Zeitpunkten t0, t der ersten und zweiten Pulswellenlaufzeitmessungen kompensiert, vorgesehen sind.

2. Vorrichtung nach Anspruch 1, **dadurch gekennzeichnet, daß** die Mittel zum Bestimmen der Pulswellenlaufzeit eine Einrichtung (45) zur Ermittlung eines Elektrokardiogramms umfassen und die Auswerteeinheit (34) geeignet ist, aus dem Elektrokardiogramm einen ersten Bezugszeitpunkt ta der Pulswellenlaufzeit zu ermitteln.

3. Vorrichtung nach Anspruch 2, **dadurch gekennzeichnet, daß** die Auswerteeinheit (34) für den ersten Bezugszeitpunkt ta die Zeit bestimmt, zu der die R-Zacke im Elektrokardiogramm auftaucht.

4. Vorrichtung nach einem der vorhergehenden Ansprüche, **dadurch gekennzeichnet, daß** die Mittel zum Bestimmen der Pulswellenlaufzeit eine Einrichtung (29) zur Detektion der Pulswellen an einer vom Herz entfernten Stelle umfassen und die Auswerteeinheit (34) geeignet ist, aus der Detektion der Pulswellen an einer vom Herz entfernten Stelle einen zweiten Bezugszeitpunkt te der Pulswellenlaufzeit zu ermitteln.

5. Vorrichtung nach Anspruch 4, **dadurch gekennzeichnet, daß** die Einrichtung (29) zur Detektion der Pulswellen an einer vom Herz entfernten Stelle eine die Pulswellen an einer Körperstelle, insbesondere an einem Finger des Patienten oder Spenders, detektierende Einrichtung ist.

6. Vorrichtung nach Anspruch 5, **dadurch gekennzeichnet, daß** die Einrichtung (29) zur Detektion der Pulswellen an einer vom Herz entfernten Stelle ein Photoplethysmograph ist.

7. Vorrichtung nach einem der vorhergehenden Ansprüche, **dadurch gekennzeichnet, daß** die Mittel (29) zur Bestimmung einer mit der Blutdichte korrelierenden Größe ein Meßgerät zur Bestimmung des relativen Blutvolumens bzw. der relativen Blutvolumenänderung umfassen und das relative Blutvolumen bzw. die relative Blutvolumenänderung die mit der Blutdichte korrelierende Größe darstellt.

8. Vorrichtung nach einem der vorhergehenden Ansprüche, **dadurch gekennzeichnet, daß** die Mittel (29) zur Bestimmung einer mit der Blutdichte korrelierenden Größe ein Meßgerät zur Bestimmung des Hämatokrits oder der relativen Hämatokritänderung umfassen und der Hämatokrit bzw. die relative Hämatokritänderung die mit der Blutdichte korrelierende Größe darstellt.

9. Vorrichtung nach einem der Ansprüche 7 oder 8, **dadurch gekennzeichnet, daß** das Meßgerät ein Ultrasschalllaufzeitmeßgerät ist.

10. Vorrichtung nach einem der Ansprüche 7 oder 8, **dadurch gekennzeichnet, daß** das Meßgerät ein optisches Durchlicht- und/oder Streulichtgerät ist.

11. Vorrichtung nach einem der vorhergehenden Ansprüche, **dadurch gekennzeichnet, daß** die Auswerteeinheit (34) eine bzgl. der Blutdichte $\rho(t0)$ zum Zeitpunkt t0 kompensierte Pulswellenlaufzeit PTT(t,p(t),$\rho$(t0)) aus dem Meßwert der Pulswellenlaufzeit PTT(t,p(t),$\rho$(t)) für den Zeitpunkt t, für die zu diesem Zeitpunkt vorliegende Blutdichte $\rho$(t) und dem vorliegenden Blutdruck p(t) nach dem folgenden Ausdruck ermittelt:

$$PTT(t, p(t), \rho(t0) = PTT(t, p(t), \rho(t)) \sqrt{\frac{\rho(t0)}{\rho(t)}} \ .$$

12. Vorrichtung nach Anspruch 11, **dadurch gekennzeichnet, daß** das Verhältnis der Blutdichten durch den Ausdruck

$$\frac{\rho(t0)}{\rho(t)} = \frac{RBV(t)}{RBV(t0)} \ ,$$

wiedergegeben wird, wobei RBV(t0) und RBV(t) die entsprechenden relativen Blutvolumina darstellen bzw. der Quotient die relative Blutvolumenänderung zwischen den Zeitpunkten t0 und t darstellt.

**13.** Vorrichtung nach Anspruch 11, **dadurch gekennzeichnet, daß** das Verhältnis der Blutdichten durch den Ausdruck

$$\frac{\rho(t0)}{\rho(t)} = \frac{HCT(t0)}{HCT(t)}$$

wiedergegeben wird, wobei HCT(t0) und HCT(t) die entsprechenden Hämatokritwerte darstellen bzw. der Quotient die relative Hämatokritänderung zwischen den Zeitpunkten t0 und t darstellt.

**14.** Vorrichtung nach einem der vorhergehenden Ansprüche, **dadurch gekennzeichnet, daß** die Vorrichtung weiterhin einen Alarmgeber (41) aufweist und die Auswerteeinheit (34) geeignet ist, anhand von hinterlegten Kriterien anormale Werte der Pulswellenlaufzeit zu erkennen und daraufhin den Alarmgeber (41) anzusteuern.

**15.** Vorrichtung nach einem der vorhergehenden Ansprüche, **dadurch gekennzeichnet, daß** die Mittel (29, 45) zum Bestimmen der Pulswellenlaufzeit gleichzeitig die Mittel (29) zur Bestimmung einer mit der Blutdichte korrelierenden Größe umfassen.

**16.** Blutbehandlungseinrichtung mit einem extrakorporalen Blutkreislauf, bestehend aus
einer Blutbehandlungseinheit (3),
einer Blutzuführleitung (5) zum Anschluß an einen Patienten oder Spender, die an einem Ende mit dem Einlaß der Blutbehandlungseinheit (3) verbunden ist und an dem anderen Ende mit dem Gefäßsystem des Patienten oder Spenders verbindbar ist,
einer Blutabführleitung (7) zum Anschluß an den Patienten oder Spender, die an einem Ende mit dem Auslaß der Blutbehandlungseinheit (3) verbunden ist und an dem anderen Ende mit dem Gefäßsystem des Patienten oder Spenders verbindbar ist,
**dadurch gekennzeichnet, daß**
die Blutbehandlungseinrichtung eine Vorrichtung (28) nach einem der vorhergehenden Ansprüche aufweist.

**17.** Blutbehandlungseinrichtung nach Anspruch 16, **dadurch gekennzeichnet, daß** die Blutbehandlungseinheit ein Hämodialysator (3) und/oder Hämofilter ist.

**18.** Blutbehandlungseinrichtung nach Anspruch 16 oder 17, **dadurch gekennzeichnet, daß** die Einrichtung zur Detektion der Pulswellen an einer vom Herz entfernten Stelle einen Meßsensor (46) umfaßt, der geeignet ist, den zweiten Bezugszeitpunkt te extrakorporal an einer Stelle des extrakorporalen Blutkreislaufs, vorzugsweise an der Blutzuführleitung (5), zu erfassen.

**19.** Blutbehandlungseinrichtung nach Anspruch 18, **dadurch gekennzeichnet, daß** der Meßsensor (46) ein Drucksensor ist.

**20.** Blutbehandlungseinrichtung nach einem der Ansprüche 16 bis 19, **dadurch gekennzeichnet, daß** die Mittel (48) zur Bestimmung einer mit der Blutdichte korrelierenden Größe extrakorporal an einer Stelle des extrakorporalen Blutkreislaufs, vorzugsweise an der Blutzuführleitung (5) vorgesehen sind.

**21.** Blutbehandlungseinrichtung nach Anspruch 20, **dadurch gekennzeichnet, daß** die Mittel (48) zur Bestimmung einer mit der Blutdichte korrelierenden Größe als optischer Durch-und/oder Streulichtsensor ausgebildet sind.

**22.** Blutbehandlungseinrichtung nach Anspruch 20, **dadurch gekennzeichnet, daß** die Mittel (48) zur Bestimmung einer mit der Blutdichte korrelierenden Größe als Ultraschalllaufzeitsensor ausgebildet sind.

**Claims**

**1.** A device for pulse-wave transit-time determination on a patient or donor, comprising means (29, 45) for determining the pulse-wave transit time of pulse waves propagating through the vascular system of a patient or donor, said pulse waves being produced by the his/her heart contractions, at a point in time t0 of a first pulse-wave transit-time measurement and at a point in time t of a second pulse-wave transit-time measurement,
**characterised in that**

means (29) for determining a quantity correlating with the blood density at points in time t0, t of the first and second pulse-wave transit-time measurements and an analyser unit (34) that compensates for the influence of the variable blood density on the first or second pulse-wave transit-time measurements with the aid of measurements of the quantity correlating with the blood density at points in time t0, t of the first and second pulse-wave transit-time measurements are provided.

2. A device according to Claim 1, **characterised in that** the means for determining the pulse-wave transit-time include a device (45) for determining an electrocardiogram and the analyser unit (34) is suitable for determining a first reference point at time ta of the pulse wave-transit time from the electrocardiogram.

3. A device according to Claim 2, **characterised in that** the analyser unit (34) determines the time at which the R wave occurs in the electrocardiogram for the first reference point at time ta.

4. A device according to one of the preceding claims, **characterised in that** the means for determining the pulse-wave transit time include a device (29) for detecting pulse waves at a site remote from the heart and the analyser unit (34) is suitable for determining a second reference point at time te of the pulse-wave transit time from detection of the pulse waves at a site remote from the heart.

5. A device according to Claim 4, **characterised in that** the device (29) for detecting the pulse waves at a site remote from the heart is a device that detects the pulse waves on a body site, in particular on a finger of the patient or donor.

6. A device according to Claim 5, **characterised in that** the device (29) for detecting the pulse waves at a site remote from the heart is a photoplethysmograph.

7. A device according to one of the preceding claims, **characterised in that** the means (29) for determining a quantity correlating with the blood density is a measurement device for determining the relative blood volume and/or a change in relative blood volume, and the relative blood volume and/or the change in relative blood volume represents the quantity correlating with the blood density.

8. A device according to one of the preceding claims, **characterised in that** the means (29) for determining a quantity correlating with the blood density comprise a measurement instrument for determining the hematocrit or the relative change in hematocrit, and the hematocrit and/or the relative change in hematocrit represents the quantity correlating with the blood density.

9. A device according to one of Claims 7 or 8, **characterised in that** the measurement instrument is an ultrasonic transit-time measurement device.

10. A device according to one of Claims 7 or 8, **characterised in that** the measurement device is an optical transmitted-light and/or scattered-light device.

11. A device according to one of the preceding claims, **characterised in that** the analyser unit (34) determines a pulse-wave transit time PTT (t, p(t), $\rho$(t0)) that is compensated with regard to the blood density $\rho$(t0) at the point in time t0 from the measured value of the pulse-wave transit time PTT (t, p(t), $\rho$(t)) for the point in time t, for the blood density $\rho$(t) prevailing at this point in time and the prevailing blood pressure p(t) according to the following equation:

$$PTT(t, p(t), \rho(t0)) = PTT(t, p(t), \rho(t)) \sqrt{(\rho(t0)/\rho(t))}$$

12. A device according to Claim 11, **characterised in that** the ratio of the blood densities is given by the equation

$$\sqrt{((\rho(t)/\rho(t0))} = \sqrt{((RBV(t0)/RBV(t))}$$

where RBV(t0) and RBV(t) denote the corresponding relative blood volumes and/or the quotient of the change in relative blood volume between points in time t0 and t.

**13.** A device according to Claim 11, **characterised in that** the ratio of the blood densities is given by the equation

$$\sqrt{(\rho(t)/\rho(t0))} = \sqrt{(HCT(t0)/HCT(t))}$$

where HCT(t0) and HCT(t) represent the corresponding hematocrit values and/or the quotient of the relative change in hematocrit between the points in time t0 and t.

**14.** A device according to one of the preceding claims, **characterised in that** the device also has an alarm-signalling device (41) and the analyser unit (34) is suitable for detecting abnormal values of the pulse-wave transit time on the basis of stored criteria and then triggering the alarm-signalling device.

**15.** A device according to one of the preceding claims, **characterised in that** the means (29, 45) for determining the pulse-wave transit time simultaneously include the means (29) for determining a quantity correlating with the blood density.

**16.** A blood-treatment machine having an extracorporeal blood circulation consisting of
a blood-treatment unit (3),
an incoming blood line (5) for connecting to a patient or donor, which line is connected at one end to the inlet of the blood treatment unit (3) and can be connected at the other end to the patient's or donor's vascular system,
an outgoing blood line (7) for connection to the patient or donor, said line being connected at one end to the outlet of the blood treatment unit (3) and being connectable at the other end to the patient's or donor's vascular system
**characterised in that**
the blood treatment machine has a device (28) according to one of the preceding claims.

**17.** A blood-treatment machine according to Claim 16, **characterised in that** the blood treatment unit is a hemodialyser (3) and/or a hemofilter.

**18.** A blood-treatment machine according to Claim 16 or 17, **characterised in that** the machine for detecting the pulse waves at a location remote from the heart includes a measurement sensor (46) that is suitable for detecting the second reference point at time te extracorporeally at a location in the extracorporeal blood circulation, preferably on the incoming blood line (5).

**19.** A blood-treatment machine according to Claim 18, **characterised in that** the measurement sensor (46) is a pressure sensor.

**20.** A blood-treatment machine according to any one of Claims 16 through 19, **characterised in that** the means (48) for determining a quantity correlating with the blood density are provided extracorporeally at a location in the extracorporeal blood circulation, preferably on the incoming blood line (5).

**21.** A blood-treatment machine according to Claim 20, **characterised in that** the means (48) for determining a quantity correlating with the blood density are designed as an optical transmitted-light and/or scattered-light sensor.

**22.** A blood-treatment machine according to Claim 20, **characterised in that** the means (48) for determining a quantity correlating with the blood density are designed as an ultrasonic transit-time sensor.

**Revendications**

**1.** Appareil pour la détermination du temps de propagation d'ondes de pouls d'un patient ou d'un donneur, comportant des moyens (29, 45) de détermination du temps de propagation d'ondes de pouls se propageant dans le système vasculaire du patient ou du donneur qui sont produites par les contractions du coeur de celui-ci, de l'instant t0 d'une première mesure de temps de propagation d'ondes de pouls à l'instant t d'une deuxième mesure de propagation d'ondes de pouls,
**caractérisé par le fait**
**qu'**il y est prévu des moyens (29) de détermination d'une grandeur en corrélation avec la densité du sang aux instants t0, t des première et deuxième mesures de temps de propagation d'ondes de pouls et

un module d'exploitation (34) qui compense l'influence de la densité variable du sang sur les première et deuxième mesures de temps de propagation d'ondes de pouls à l'aide des mesures de la grandeur en corrélation avec la densité du sang aux instants t0, t des première et deuxième mesures de temps de propagation d'ondes de pouls.

2. Appareil selon la revendication 1, **caractérisé par le fait que** les moyens de détermination du temps de propagation d'ondes de pouls comprennent un dispositif (45) pour l'établissement d'un électrocardiogramme, et le module d'exploitation (34) est apte à déterminer à partir de l'électrocardiogramme un premier instant de référence ta du temps de propagation d'ondes de pouls.

3. Appareil selon la revendication 2, **caractérisé par le fait que** le module d'exploitation (34) détermine pour le première instant de référence ta le temps auquel le pic R apparaît dans l'électrocardiogramme.

4. Appareil selon l'une des revendications précédentes, **caractérisé par le fait que** les moyens de détermination du temps de propagation d'ondes de pouls comprennent un dispositif (29) pour la détection des ondes de pouls à un endroit éloigné du coeur, et le module d'exploitation (34) est apte à déterminer à partir de la détection des ondes de pouls à un endroit éloigné du coeur un deuxième instant de référence te du temps de propagation d'ondes de pouls.

5. Appareil selon la revendication 4, **caractérisé par le fait que** le dispositif (29) pour la détection des ondes de pouls à un endroit éloigné du coeur est un dispositif qui détecte les ondes de pouls à un endroit du corps, en particulier sur un doigt du patient ou du donneur.

6. Appareil selon la revendication 5, **caractérisé par le fait que** le dispositif (29) pour la détection des ondes de pouls à un endroit éloigné du coeur est un photopléthysmographe.

7. Appareil selon l'une des revendications précédentes, **caractérisé par le fait que** les moyens (29) de détermination d'une grandeur en corrélation avec la densité du sang comprennent un appareil de mesure pour la détermination du volume sanguin relatif ou de la variation relative du volume sanguin, et le volume sanguin relatif ou la variation relative du volume sanguin représente la grandeur en corrélation avec la densité du sang.

8. Appareil selon l'une des revendications précédentes, **caractérisé par le fait que** les moyens (29) de détermination d'une grandeur en corrélation avec la densité du sang comprennent un appareil de mesure pour la détermination de l'hématocrite ou de la variation relative de l'hématocrite, et l'hématocrite ou la variation relative de l'hématocrite représente la grandeur en corrélation avec la densité du sang.

9. Appareil selon l'une des revendications 7 et 8, **caractérisé par le fait que** l'appareil de mesure est un appareil de mesure de temps de propagation d'ultrasons.

10. Appareil selon l'une des revendications 7 et 8, **caractérisé par le fait que** l'appareil de mesure est un appareil optique à transmission ou à diffusion de lumière.

11. Appareil selon l'une des revendications précédentes, **caractérisé par le fait que** le module d'exploitation (34) détermine un temps de propagation d'ondes de pouls PTT(t, p(t), ρ(t0)) compensé relativement à la densité du sang ρ(t0) à l'instant t0 à partir de la valeur mesurée du temps de propagation d'ondes de pouls PTT(t, p(t), ρ(t)) pour l'instant t, pour la densité du sang ρ(t) existant à cet instant et la tension artérielle existante p(t), suivant l'expression :

$$PTT(t, p(t), \rho(t0)) = PTT(t, p(t), \rho(t))\sqrt{\frac{\rho(t0)}{\rho(t)}}$$

12. Appareil selon la revendication 11, **caractérisé par le fait que** le rapport des densités du sang est donné par l'expression

$$\frac{\rho(t0)}{\rho(t)} = \frac{RBV(t)}{RBV(t0)},$$

RBV (t0) et RBV (t) représentant les volumes sanguins relatifs correspondants, et le quotient représentant la variation relative du volume sanguin entre les instants t0 et t.

13. Appareil selon la revendication 11, **caractérisé par le fait que** le rapport des densités du sang est donné par l'expression

$$\frac{\rho(t0)}{\rho(t)} = \frac{HCT(t0)}{HCT(t)}$$

HCT (t0) et HCT (t) représentant les valeurs d'hématocrite correspondantes, et le quotient représentant la variation relative de l'hématocrite entre les instants t0 et t.

14. Appareil selon l'une des revendications précédentes, **caractérisé par le fait qu'**il comprend en outre un émetteur d'alarme (41) et que l'appareil d'exploitation (43) est apte à déceler à l'aide de critères consignés des valeurs anormales du temps de propagation d'ondes de pouls et ensuite déclencher l'émetteur d'alarme (41).

15. Appareil selon l'une des revendications précédentes, **caractérisé par le fait que** les moyens (29, 45) de détermination du temps de propagation d'ondes de pouls comprennent en même temps les moyens (29) de détermination d'une grandeur en corrélation avec la densité du sang.

16. Dispositif de traitement de sang comprenant une circulation sanguine extracorporelle, constitué d'un appareil de traitement de sang (3),
d'une conduite d'arrivée de sang (5) destinée à être appliquée sur un patient ou un donneur qui est connectée à une extrémité à l'entrée de l'appareil de traitement de sang (3) et peut être connectée à l'autre extrémité au système vasculaire du patient ou du donneur,
d'une conduite de départ de sang (7) destinée à être appliquée sur le patient ou au donneur qui est connectée à une extrémité à la sortie de l'appareil de traitement de sang (3) et peut être connectée à l'autre extrémité au système vasculaire du patient ou du donneur.,
**caractérisé par le fait qu'**il présente un appareil (28) selon l'une des revendications précédentes.

17. Dispositif de traitement de sang selon la revendication 16, **caractérisé par le fait que** l'appareil de traitement de sang est un hémodialyseur (3) et/ou un hémofiltre.

18. Dispositif de traitement de sang selon l'une des revendications 16 et 17, **caractérisé par le fait que** le dispositif de détection des ondes de pouls à un endroit éloigné du coeur comprend un capteur de mesure (46) qui est apte à saisir le deuxième instant de référence te extracorporellement à un endroit de la circulation sanguine extracorporelle, de préférence sur une conduite d'arrivée de sang (5).

19. Dispositif de traitement de sang selon la revendication 18, **caractérisé par le fait que** le capteur de mesure (46) est un capteur de pression.

20. Dispositif de traitement de sang selon l'une des revendications 16 à 19, **caractérisé par le fait que** les moyens (48) de détermination d'une grandeur en corrélation avec la densité du sang sont prévus extracorporellement à un endroit de la circulation sanguine extracorporelle, de préférence sur une conduite d'arrivée de sang (5).

21. Dispositif de traitement de sang selon la revendication 20, **caractérisé par le fait que** les moyens (48) de détermination d'une grandeur en corrélation avec la densité du sang sont constitués d'un capteur optique de lumière transmise et/ou de lumière diffuse.

**22.** Dispositif de traitement de sang selon la revendication 20, **caractérisé par le fait que** les moyens (48) de détermination d'une grandeur en corrélation avec la densité du sang sont constitués d'un capteur de temps de propagation d'ultrasons.

# Fig. 1

Fig. 2

EP 1 199 029 B1